# EUROPEAN PATENT APPLICATION

(11) **EP 4 552 668 A1**
(43) Date of publication of application: **14.05.2025**
(21) Application number: 24211396.7
(22) Date of filing: 07.11.2024
(51) Int. Cl.: A61M 5/142

(54) **IMPROVED AUTOINJECTOR ASSEMBLY**

(30) Priority: 07.11.2023 US 202363596820 P
(71) Applicant: Critical Innovations, LLC, Lawndale, CA 90260 (US)
(72) Inventor: DONALDSON, Ross I., Lawndale, CA 90260 (US); YESAYAN, Zhirair, Lawndale, CA 90260 (US); SANCHEZ, Steve, Lawndale, CA 90260 (US); BUCHANAN, Oliver, Lawndale, CA 90260 (US); SCAGLIONE, Bernard, Lawndale, CA 90260 (US); INGAMELLS, Grant, Lawndale, CA 90260 (US); YOUNG, Tyler, Lawndale, CA 90260 (US)
(74) Representative: HGF

(57) **Abstract**

An improved method and device for delivering one or more medicinal fluid and/or drug to the body. The device generally comprises an improved autoinjector assembly. The provided improved autoinjector assembly substantially improves injection of one or more medicinal fluid and/or drug to the body.

## Description

### Priority Claim

This application claims the benefit of U.S. Provisional Application No. 63/596,820, filed November 7, 2023, which is hereby incorporated by reference herein in its entirely.

### Commonly Owned Applications

U.S. Patent Application Ser. 13/961,422, filed August 7, 2013 and entitled Method and Device for Simultaneously Documenting and Treating Tension Pneumothorax and/or Hemothorax; U.S. Patent Application Ser. No. 14/581,339, filed December 23, 2014 entitled Percutaneous Channel System and Method; U.S. Patent Application Ser. No. 16/113,707, filed August 27, 2018 and titled Percutaneous Access Pathway System; and U.S. Patent Application Ser. No. 16/354,418, filed March 15, 2019 and titled Systems and Methods Relating to Medical Applications of Synthetic Polymer Formulations, U.S. Patent Application Ser. No. 16/948,885, filed October 5, 2020 and entitled Percutaneous Access Pathway System, U.S. Patent Application Serial No. 17/876,187, filed July 28, 2022 and entitled Wound Treatment Device, U.S. Patent Application Serial No. 18/448,455, filed August 11, 2023 and entitled Percutaneous Access Pathway System, and U.S. Patent Application Serial Number 18/485,800, filed October 12, 2023 and entitled Systems and Methods Relating to Medical Applications of Inverse Thermosensitive Polymer Foam Formulations are hereby incorporated by reference herein in their entirety.

This invention was made with government support under contract HT9425-23-C-0 11, "Rx-Shooter Wearable Wound Infection Treatment Device," awarded by U.S. Army Medical Research Acquisition Activity (USAMRAA). The government has certain rights in the invention.

### Technical Field

The present disclosure relates generally to the field of medical devices, and more particularly, to devices and methods for delivering one or more medicinal fluids and/or drugs to the body.

### Background

Despite major advancements in military medicine, in recent wars including Iraq and Afghanistan more than one-third of patients with a combat injury evacuated to Continental United States hospitals had an infection diagnosed during their initial hospitalization. Open combat wounds are prone to infection, particularly in the far forward environment, and they can be a late cause of morbidity and mortality in combat casualties. To prevent the development of infection, current Tactical Combat Casualty Care (TCCC) guidelines recommend antibiotic treatment for open wounds sustained on the battlefield. When indicated, antibiotics should be administered immediately after trauma, with delays of even one or two hours significantly increasing infection rates. However, there is a gap between recommendation and reality, as the currently available far forward treatment for infection prevention is infrequently utilized.

Presently, troops are issued a combat wound medication pack (CWMP), which can be found in the Individual First Aid Kit (IFAK). The CWMP contains: 2 (650 mg) tablets Acetaminophen, 1 (400 mg) tablet Moxifloxacin, and 1 (15 mg) tablet Meloxicam. TCCC-recommended point of injury care for open wounds includes administration of antibiotic Moxifloxacin (400 mg, PO once a day) from the CWMP. However, recent research shows that less than one percent of casualties who met criteria for CWMP administration received the intervention. Compliance is low for many reasons, including lack of tolerance for oral administration. If a casualty is unable to swallow due to shock or loss of consciousness, the TCCC recommendation is to refer to a medic for intravenous (IV) administration. This is problematic in the far forward environment, as rapid IV administration may be hindered by personnel, materiel, and time requirements. This is also true for civilian patients treated by traditional Emergency Medical Services (EMS). Additionally, multi-domain operations may further hinder management of such injuries on the future battlefield. An initial responder, forward-deployable treatment for infection prevention after such injuries would be a major advance for military combat care during prolonged and *en route* care in austere and combat environments, while additionally providing benefit in civilian trauma scenarios.

An autoinjector is a medical device designed to deliver a dose of a particular drug into the body of a patient (e.g., injecting oneself or another person). Typically, an autoinjector simplifies and/or facilitates at least one step in the process of injecting a drug, in comparison to manual drug injection by a healthcare provider. This facilitates self-administration or administration by untrained or lesser-trained personnel. Classically, autoinjectors have a single preloaded dose of a single drug that typically is delivered via a pen-like spring-loaded syringe that is activated when the device is pushed firmly against the body. However, the literature describes a wide range of art related to autoinjectors and related on-body injectors.

Autoinjectors and related technology have the potential to overcome the challenges associated with the CWMP and more traditional means of IV administration, providing a delivery method that does not require oral administration and is simple enough to be used by self- or buddy-aid. Although the current state of autoinj ector technology is limited for adequate combat and many cases of civilian use.

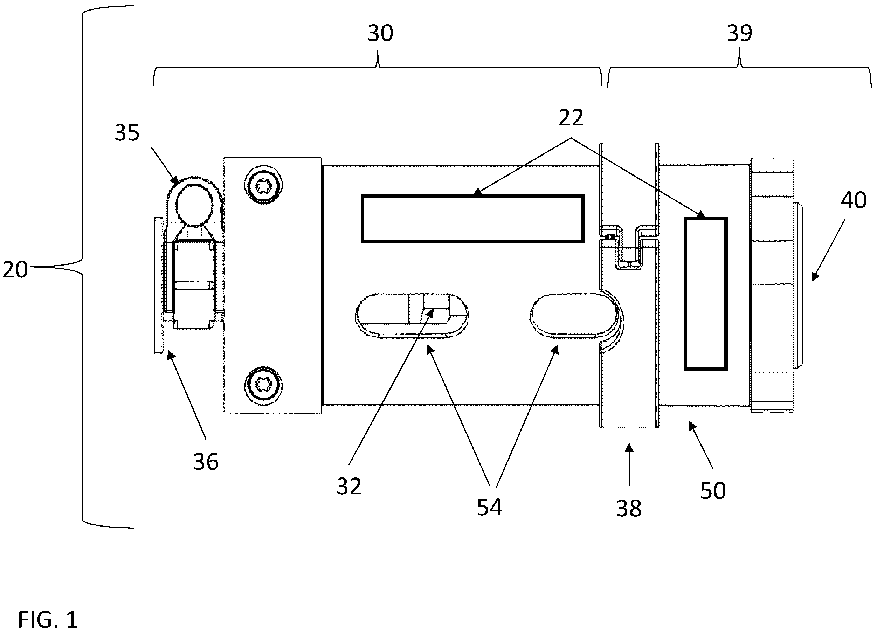

Despite the availability of autoinjectors and their ease of use, there are numerous drawbacks with current autoinjector technology. First, with the exception of the Antidote Treatment, Nerve Agent, Autoinjector (ATNAA), most autoinjectors are for a single drug, each coming in a large pen-marker-like size. This also includes autoinjectors for naloxone (e.g., for opioid overdoses) and epinephrine (e.g., for anaphylaxis). Carrying such devices, especially in cases where repeat doses are likely to be necessary, adds size and weight to the already overloaded warfighter. Additionally, there are multiple other drugs that might be of use that are currently not available in autoinjector form. Figure 1 shows examples of drugs given by autoinjector. To our knowledge, there is currently no antibiotic available as an autoinjector approved for use by the FDA.

Second, autoinjectors are not adapted for use in the far forward environment. Pen autoinjectors are not shock proof and can be limited to 1 meter drop shock. Additionally, some devices for drugs with higher viscosity have issues with pre-activation or glass breakage due to overpowering spring-driven systems. This means that even though autoinjectors have the potential to quickly and easily deliver life-saving treatment on the battlefield, existing devices are not adequately built to withstand its unique challenges.

Third, such autoinjectors all deploy via traditional intramuscular injection that must be given in very specific parts of the body to avoid important underlying structures (e.g., larger veins, arteries, and nerves). This frequently requires additional training requirements to ensure appropriate use and the minimization of complications.

Insulin pumps for diabetic patients are currently the main devices cleared by the FDA for human use to inject medicinal fluids into the body. There are two microneedle-patch insulin pumps approved for sale in the United States (Omnipod Insulin Management System from Insulet and V-Go from Valeritas), as well as an additional system approved in Europe (Accu-Chek Solo by Roche) [7] [8]. Around the size of a deck of cards and with drug reservoirs holding up to 2-4 mL, these are worn directly on the body via an adhesive patch (i.e., no traditional pump tubing). Both the Omnipod and Accu-Check can be worn for several days straight, with the Omnipod additionally being waterproof up to 25 feet deep for 60 minutes. However, these pumps and related technology are not directly transferable to the combat environment because they are 1) cartridge-based systems that require planetary gearing with limited power output due to battery power and motor sizing; 2) single use with no way to deliver without battery power; and/or 3) expensive due to the use of technology that is focused on giving a titration over time, as opposed to much less expensive bolus dosing that is the standard for most drug delivery.

Microneedle technology has recently been integrated into a "peel & stick" format, such as the patch developed by Micron Biomedical. Micron's microneedle patch is applied by pressing it against the skin, allowing for easy self-administration. Upon application of the patch to the skin, polymer microneedles penetrate the upper layers of skin and then rapidly dissolve, releasing their drug(s). After a few minutes, the patch is removed from the skin. While this technology has benefits regarding portability and ease of use, the system has fundamental limitations. First, the drugs that can be delivered via this system are limited to those that have been specifically formulated to be compatible with the dissolving microneedle technology, which significantly limits its benefit for the warfighter. Second, there is no way for this system to deliver automated repeat dosing over time. Instead, the user must manually deploy treatment when required. This functionally limits the technology in the hectic combat environment to single dose use and not practical for longer term care (e.g., PFC of up to 72 hours). Finally, there is limited volume that can be delivered intradermally, which significantly limits the types of antibiotics and other pharmaceuticals that can be delivered through this vehicle.

Fourth, many autoinjectors store the drug as a fluid, which limits drug stability and the library of drugs that can be delivered using the device. Lyophilized or otherwise dried product (i.e. medicament) can in many cases be stored for longer periods of time and then later combined with a fluid (i.e., diluent). Reconstitution means that a powder (e.g., lyophilized freeze-dried drug) is mixed with a diluent (e.g., liquid). Diluents frequently do not contain any pharmacological action but are necessary for reconstituting drugs. Commonly used solvents used in diluting solutions include, but are not limited to, sterile water, bacteriostatic water, water for injection, bacteriostatic sodium chloride 0.9%, and sodium chloride 0.9%. The prior art, for example US 10,226,583 to Edwards et al., includes autoinjectors wherein a diluent can be stored separately from a medicament and upon actuation mixed such that the combination of the diluents and the medicament reconstitute the medicament for delivery. Such auto-injectors are often referred to as "wet/dry" auto-injectors, because one medicament is often a liquid (e.g., water or another diluent) and the other medicament can be substantially solid or dry (e.g., drug powder). In use, the first medicament and the second medicament must be mixed prior to injection.

Fifth, the site selection for delivery of most current autoinjectors is done by the end user. There does not currently exist autoinjectors where a separate provider can identify an on-board position for multi-dose delivery of an injection that a user can later use.

Sixth, although wearable autoinjectors exist, the prior art for these wearable autoinjectors does not integrate with discrete and changeable medication capsules that contain the drug of interest and automatic sharps protection before and after delivery.

In the civilian market, parenteral antibiotics are sometimes needed due to their beneficial properties over oral antibiotics. In these cases, under current care, patients frequently need to be admitted to the hospital, started on parenteral antibiotics (frequently via IV), have a PICC line placed, and then transition to home care with a nurse injecting through the intravenous PICC line.

The literature discloses various additional known methods and devices related to autoinjectors. However, all are limited in some aspect.

Each of the patents and published patent applications mentioned above are hereby incorporated by reference.

### Summary

The present disclosure overcomes and substantially alleviates the deficiencies in the prior art by providing improved devices and methods related to delivering one or more medicinal fluids and/or drugs to the body. Under various embodiments, an improved autoinjector assembly is formed via different methods and devices, which include the aforementioned techniques noted as background of the present disclosure and that are incorporated by reference. The provided improved autoinjector assembly substantially improves injection of one or more medicinal fluid and/or drug to the body.

Under embodiments, improved autoinjector assembly technology provides a mechanized injection delivery system that can rapidly and accurately administer one or more medications. One aspect of the present disclosure provides an improved autoinjector assembly that includes one or more drug capsules and one or more drug delivery holders. An object of this system is to allow for easy interoperability between drug capsules and delivery holders, such that individual drug capsules (e.g., with the same type of drug and/or with different types of drugs) can all work with different drug delivery holders (e.g., with the same type of drug delivery holder and/or with different types of drug delivery holders).

Under embodiments, each drug capsule is single-use, delivers a fixed-dose, and is color-coded by medication for easy identification. It has clear, easy-to-read labeling to allow for quick selection of the desired medication via selecting the appropriate drug capsule. Under embodiments, the drug capsule not only includes its individual drug, but also at least partially the means for delivering that drug to the body. For example, each medication can have different properties when mixed with a diluent, such as viscosity, that require tailoring components of an autoinjector to delivery of that drug. Under embodiments, these medication-specific modifications are all encompassed within the drug capsule itself, thus allowing the drug delivery holder to not need modification based on drug capsule used in conjunction with it. Under embodiments, each drug capsule includes at least one drug for injection, at least part of the injection mechanism, and a needle.

Under embodiments, drug capsules have safety features such that they can be safely carried and handled by the user without concern for sterility or sharps risk. Under embodiments, this includes a drug capsule cap that functions in conjunction with the rest of the drug capsule prior to use to prevent damage from the external environment, prevent premature discharge during storage, and/or maintain sterility of at least some area of the drug capsule. Under embodiments, once the drug capsule cap is removed, the drug capsule has additional safety features that prevent its premature discharge until connected and/or placed into a drug delivery holder. This improved system allows even minimally-trained users to easily load drug capsules into the drug delivery holder, thus facilitating its use. Under embodiments, drug capsules additionally include a viewing window or other related means for visualizing the drug prior to drug capsule use (e.g., to confirm that the medication is fully dissolved in solution, to confirm that the medication is unadulterated, to confirm that the medication was fully delivered, etc.). Under embodiments, the drug capsule includes information specific to the contained drug (e.g., dose, medication type, indications, contraindications, side effects, use instructions).

Under embodiments, once placed into a drug delivery holder and only once placed with such a holder, the drug capsule may be triggered to deliver the drug to the body. Under embodiments, an individual who is not the principal user can place the holder prior to the user using the rest of the system (e.g., a healthcare provider may place the holder in its correct anatomical position, so that a patient-user does not need to determine the appropriate injection location). Under embodiments, device does not require nor include a drug delivery holder. Under embodiments, the drug delivery holder can hold more than one drug capsule. Examples include the serial insertion of drug capsules into a given capsule receiving area, the parallel insertion of drug capsules into multiple different capsule-receiving areas, and/or a combination of the two. Under embodiments, the drug delivery holder is at least partially reusable, while each drug capsule is single-use and disposable after use. Under embodiments, the drug capsule's single-use inner sterile needle is caused to exit the drug capsule through a sterile barrier specific to the drug capsule and subsequently penetrate the patient. Under embodiments, the improved autoinjector assembly then causes a liquid medication from its drug storage chamber to be injected through one or more hollow needles.

Under embodiments, drug injection is actuated by the release of pressurized gas to actuate needle insertion and drug delivery in a sequential process. Under other embodiments, at least part of this process is actuated by use of one or more springs or other resilient biasing mechanisms.

Under embodiments, the improved autoinjector assembly delivers drug(s) through different routes. For example, under some embodiments the needle is of appropriate structure to deliver intradermal (ID), subcutaneous (SQ), intramuscular (IM), and/or intravenous (IV). Under various embodiments the needle delivers to an injection depth ranging from 0-0.5 cm, 0.5-1 cm, 1-1.5 cm, 1.5-2 cm, 2-2.5 cm, 2.5-3 cm, 3-3.5 cm, 3.5-4 cm, 4-4.5 cm, 4.5-5 cm, and/or 5-10 cm. In some embodiments, SQ injection is used to minimize the risk to underlying structures (e.g., larger arteries, veins, nerves, and other significant tissues), while still allowing multiple places for device placement over the body. Under embodiments, drug capsules may allow for the injection of different volumes (e.g., about 0.5 mL, about 1 mL, about 1.5 mL, about 2 mL, about 2.5 mL, about 3 mL, about 3.5 mL, about 4 mL, about 4.5 mL, about 5 mL, >5 mL).

Under embodiments, once the drug is delivered, the improved autoinj ector assembly causes the one or more needles to retract back into the drug capsule. Under embodiments, after drug delivery the counterforce from an internal spring causes the needle to retract back into the drug capsule. Under embodiments, this mechanism minimizes the time the needle is in the body, thus reducing pain from needle penetration and complication risk. Under embodiments, this prevents and/or reduces the chances of needle stick injury by automatically causing the used drug capsule to serve as a sharp-protecting container. With the hazardous needle retracted back into the drug capsule, the drug capsule can then be removed from the drug delivery holder for safe disposal. Under embodiments, actuation causes a visual indicator on the drug capsule to show that it has been used, to avoid confusion with unused drug capsules. Under embodiments, used drug capsules are permanently changed to indicate previous use and/or physically changed to prevent reuse. This provides benefits regarding concern for subsequent needle stick injuries and/or biohazard contamination.

Under embodiments, drug delivery holders come in different sizes and shapes. Under embodiments, the drug delivery holder is an off-body system that functions to allow use of a drug capsule on a patient (e.g., the combination of a drug capsule and drug delivery holder form a pen or gun-like assembly for drug delivery). Under embodiments, the drug deliver holder is an on-body system that can stay strapped to a patient while one or more drug capsules are used. Examples include different versions of securely attaching the device to the body, including a Velcro-enabled strap, other straps, skin safe adhesive pad, and/or integration into the uniform itself. Under embodiments, the drug delivery holder accepts one, two, three, four, five, six, or more drug capsules at a time. Under embodiments, the drug delivery holder accepts only a single drug capsule and is used serially to deliver medications one-at-a-time. Under embodiments, a larger drug delivery holder accepts multiple drug capsules at the same time. For example, multiple drug capsules of the same or different medication type could be inserted into a drug delivery holder and the device used to delivered them over a set period (e.g., every twelve hours over three days). Under embodiments, drug capsule activation is manual, automated, or a combination of the two.

Under embodiments, the improved autoinjector assembly facilitates the administration of one or more therapeutics by a patient and/or lesser-skilled user, rather than a trained provider. For example, the drug delivery holder could be positioned initially by a healthcare provider to ensure that injections are made in the correct location. Then, the patient could be sent home and the patient and/or a family member could then use the device to make injections over some time course unsupervised by the healthcare provider. This is an improvement on current autoinjector technology, which requires the patient to identify the appropriate anatomic location for every injection.

Under embodiments, the improved autoinjector assembly additionally includes built-in sensors and/or connection to external sensors and/or communications infrastructure. For example, under some embodiments the system electronically pairs with biometric sensors and, when triggered, automatically delivers one or more selected drugs as appropriate. Embodiments allow the possibility of manual, semi-automatic, and/or automatic triggering via different routes such as self-administration, buddy care, local provider use, and/or remote telemedicine. Under embodiments, this includes connection to artificial intelligence and/or machine learning algorithms.

Under embodiments, an improved autoinjector is tailored for use in the out-of-hospital environment (e.g., far forward combat, emergency medical services, in-home, home health, nursing care), while in others it is tailored to the in-hospital environment. Under embodiments, this assembly is robust, reliable, compact, lightweight, cost effective, and/or affordable; suitable for use across a wide range of temperature and operational environmental conditions; simple to operate for self-, buddy, and provider assisted-aid; and/or quick in onset of action. Under embodiments, improved autoinjector assembly is designed to be comfortably worn by a warfighter under, over, or as part of a uniform and/or protective gear.

Under embodiments, the improved autoinjector assembly uses modular drug capsule technology to provide the ability to deliver an interoperable library of medications in the form of different drug capsules. Under embodiments, the improved autoinjector assembly can deliver repeat drug dosages over time. Under embodiments, the improved autoinjector assembly can be used only for the time it takes to actuate the system and deliver a drug, for hours, for days, for weeks, and/or for months. These benefits are in comparison to traditional autoinjectors that only facilitate a single injection at a single time to an identified anatomic area. Under some embodiments, the drug delivery holder is maintained on the body for 7-10 days while additional drug capsules may be inserted into it as needed.

Under embodiments, the improved autoinjector assembly meets one or more of the following operational requirements: the drug capsule must not activate outside of the drug delivery holder; the improved autoinjector assembly must only activate after direct manual input; the autoinjector assembly must be operational after exposure in temperature range from 5C to 40C; the autoinjector assembly must be operational after experiencing a one (1) meter drop; it must be easy to insert the drug capsule into the drug delivery holder; it must be easy to remove the drug capsule from the drug delivery holder; the drug capsule must not fall out of the drug delivery holder by itself; the therapeutic must be visible inside the drug capsule before insertion into the drug delivery holder; the therapeutic must be protected within the drug capsule from light when stored; the drug capsule must be sealed in a container prior to being readied for the drug delivery holder; and/or the patient must not suffer an adverse reaction from receiving multiple injections in the same location.

Under embodiments, the improved autoinjector assembly meets one or more of the following physical requirements: the drug capsule diameter is less than 55 mm; the drug capsule total height profile is less than 68 mm; the drug capsule total mass when full to required maximum volume is less than 50 g; the drug capsule delivers between 0.3 mL and 2.0 mL of drug product solution, excluding hold up volume (H.U.V.); and/or the markings on the drug capsule, drug delivery holder, and/or autoinjector assembly are legible within luminated conditions 100 lx at 30cm to 70cm distance.

Under embodiments, the improved autoinjector assembly meets one or more of the following performance requirements: the improved autoinjector assembly allows strap drug delivery holder placement on either arm and/or thigh; the drug delivery holder is able to remain in place for 7-10 days; after placement, the drug delivery holder must hold securely to its starting location; the delivery of the therapeutic drug must be automatic after manual activation; the improved autoinjector assembly must indicate that it is ready for delivery; the improved autoinjector assembly's undeployed state should clearly differ from its deployed state; the improved autoinjector assembly and/or its components must withstand a 1-meter drop; and/or the drug capsule needle should be guarded before and after the dose has been delivered.

Under embodiments, the improved autoinjector assembly meets one or more of the following other requirements: improved autoinjector assembly and/or its components will be provided sterile; improved autoinjector assembly and/or its components should not be re-sterilized; the autoinjector assembly in its terminal packaging will be designed to withstand exposure to any environmental insults that may reasonably be expected during air or ground shipping within the US and to international locations; the improved autoinjector assembly will remain stable when stored at temperatures from 15-30°C; the expiration date for the device will be equal to or greater than 12 months from the date of manufacture; the autoinjector assembly system is for single-patient use and should be disposed of in accordance with Federal, State, and Local Regulations for sharps and infectious materials disposal; the application of the improved autoinjector assembly should be simple, deploy quickly, and with minimal steps; and/or the application of the device should be possible with the patient standing, sitting, or lying down.

The improved autoinjector assembly can deliver one or more medicinal fluids and/or drugs to the body (i.e., drug, therapeutic, medicine). In embodiments, these include all existing types of standard pharmaceutical formulations, as well as the adjustment of such formulations for longer storage and/or use with the improved autoinjector assembly. Under embodiments, the autoinjector assembly includes one or more therapeutics. Under embodiments, the assembly includes a library of different injectable drug products. Although not limited to these categories and drugs, examples include antibiotics (e.g., cefazolin, ceftriaxone, ertapenem, levofloxacin, teicoplanin), analgesic agents (e.g., fentanyl, hydromorphone, ketamine, ketorolac, morphine), anticonvulsants (e.g., diazepam, lorazepam, midazolam), antiemetics (e.g., metoclopramide, promethazine), cardiac / ACLS medications (e.g., epinephrine), chem bio defense medications (e.g., 2-PAM, atropine), opioid antagonists (e.g., naloxone), and/or numerous others (e.g., diphenhydramine, insulin, tranexamic acid).

Under some embodiments, the drug to be delivered is within a liquid solution prior to delivery. Under some embodiments, the drug to be delivered is stored in a dry form (e.g., lyophilized) prior to initiating the delivery process. This has the added benefit of increasing storage time and environmental stability. In some embodiments where the drug is in a dry form, there is an additional reconstitution step (e.g., automated or manual) that causes the dry drug to first mix with a fluid before injection. For example, in embodiments, the drug capsule cap and/or another part of the drug capsule contains a diluent in a diluent chamber. A diluent actuator causes the diluent to mix with the dry drug. The user can then view the combined product through a viewing window in the drug capsule and can shake the drug capsule to cause the drug to go into solution. Once combined, the user can then insert the drug capsule into the drug delivery holder to deliver the medication as elsewhere described.

Under embodiments, one or more of the areas and/or components of the device is provided sterile to the user. Under embodiments, one or more layers of packaging maintains the sterility of the device (e.g., prevents contamination from the outer environment). Under embodiments, one or more of the following methods are used for sterilization of the one or more of the areas and/or components of the device: steam sterilization, ethylene oxide, peracetic acid, dry heat, radiation (e.g., gamma ray, electron beam, X-ray), and/or hydrogen peroxide (e.g., vaporized, gas plasma). Under embodiments, sterilization is performed terminally and/or in process.

There have been illustrated and described herein methods and devices related to autoinjectors. While particular embodiments of the disclosure have been described, it is not intended that the disclosure be limited thereto, as it is intended that the disclosure be as broad in scope as the art will allow and that the specification be read likewise.

From the foregoing, it can be seen that the present disclosure provides improved means for auto-injection within animals, especially humans. In various embodiments, the device is used for auto-injection into different body areas. These include intradermal, subcutaneous, intramuscular, intravenous, and specific structures (e.g., intraarticular, intracavitary, intra-central nervous system). Although the example subcutaneous injection has at times been used to illustrate the disclosure, it should not limit its scope as it could also similarly be used to access anatomical locations.

Moreover, it should also be apparent that the device can be made in varying lengths, sizes and capacities, and the precise composition of the device may be varied appropriately to treat adults, children, and infants. While the device has been described with a certain degree of particularity, it is manifest that many changes may be made in the details of construction and the arrangement of components without departing from the spirit and scope of this disclosure. It is understood that the disclosure is not limited to the embodiments set forth herein for purposes of exemplification and that elements of certain embodiments can be combined with elements of other embodiments. Additional objects, advantages, and novel features of the disclosure will be set forth in the description which follows, and will become apparent to those skilled in the art upon examination of the following detailed description and figures. It should be understood that not all of the features described need be incorporated into a given system or method.

### Brief Description of the Drawings

FIG. 1 is an isometric view of an improved autoinjector assembly in accordance with an embodiment of the disclosure.
FIG. 2 is a cross-sectional view of an improved autoinjector assembly in accordance with an embodiment of the disclosure.
FIG. 3 is a cross-sectional view of an improved autoinjector assembly in accordance with an embodiment of the disclosure.
FIG. 4 is a cross-sectional view of an improved autoinjector assembly in accordance with an embodiment of the disclosure.
FIG. 5 is a cross-sectional view of an improved autoinjector assembly in accordance with an embodiment of the disclosure.

### Detailed Description

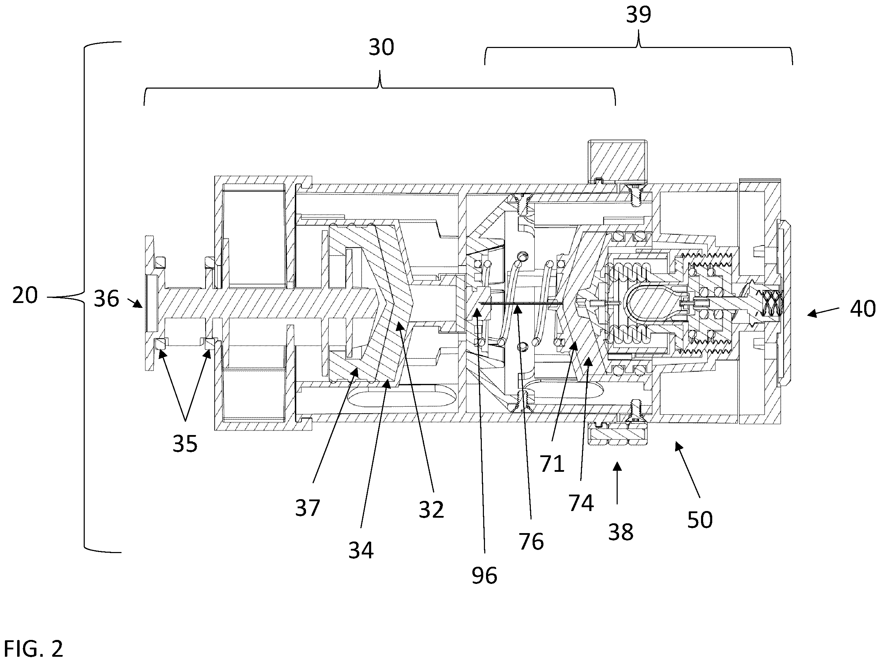

Referring to the drawings, FIGS. 1-2 illustrate one embodiment of part of the present disclosure. For ease of reference, distal shall refer to the end of the device farthest away from the user, while proximal shall refer to the end of the device closest to the user. Under this embodiment, an improved autoinjector assembly is shown, including drug capsule 20. In embodiments, drug capsule 20 includes drug capsule cap 30 and drug capsule container 39. In embodiments, drug capsule cap 30 further includes drug capsule actuator 40 and drug capsule body 50. In embodiments, drug capsule 20 additionally contains one or more drug information area(s) 22, which provide capsule-specific drug information to allow the user to easily identify the drug contained within the capsule and/or other applicable details (e.g., dosage, frequency of delivery, medication type). In embodiments, cap connector 38 prevents removal of drug capsule cap 30 from drug capsule container 39 until desired by the user and/or allowed by the device. Before removal, drug capsule cap 30 and/or cap connector 38 can serve to protect the sterile distal area of drug capsule 20 (e.g., during transport and/or handling) and/or maintain its sterility. In embodiments, removal of drug capsule cap 30 from the rest of drug capsule 20 is prevented by cap connector 38. In embodiments, removal of cap connector 38 is performed manually by the user. In embodiments, cap connector 38 is a mechanism internal to the device that is only able to be manually, semi-automatically, and/or automatically disconnected after diluent has successfully been transferred from capsule cap 30 to drug capsule container 39.In embodiments, drug capsule cap 30 facilitates charging of the system with diluent 32, for example causing the initially separately stored fluid diluent 32 to move into injection chamber 74 and mix with dry (e.g., lyophilized) drug 71, thus becoming a mixture capable for injection (e.g., reconstitution). In embodiments, the user first removes or otherwise disengages diluent actuator lock 35, which then allows movement of diluent actuator 36. In embodiments, diluent actuator 36 is similar to a standard syringe plunger system and connected to rubber stopper 37 located within diluent chamber 34. In the shown embodiment, the user then pushes diluent actuator 36 distally, which causes patient needle 76 to pierce needle septum 96 to make a contiguous space with diluent chamber 34 and thus allow diluent 32 to travel into drug capsule container 39 and injection chamber 74. In embodiments, there is a locking mechanism (not shown) that prevents diluent actuator 36 from subsequently retreating proximally once diluent has been sufficiently transferred to drug capsule container 39. In embodiments, there is a disconnecting mechanism (not shown) that disengages and/or allows the disengagement of cap connector 38 once and only once diluent has been sufficiently transferred to drug capsule container 39.

In embodiments, drug capsule cap 30 cannot be removed from drug capsule container 39 until drug capsule container 39 is sufficiently charged with fluid (e.g., prevented by an interaction with cap connector 38). In embodiments, cap connector 38 is manually removed after the system is charged. In embodiments, charging of the system occurs automatically when removing drug capsule cap **30** from the rest of drug capsule container **39.** In embodiments, the user shakes and/or agitates drug capsule cap **30** and/or drug capsule container **39** to facilitate dissolving drug **71** into diluent **32** to form a combined solution. In embodiments, one or more viewing windows **54** allow the user to view at least part of injection chamber **74** from the outside of drug capsule container **39.** This can allow viewing to see if sufficient drug has dissolved into liquid solution before deployment and/or to determine if sufficient liquid and/or drug product has been injected into the patient post-actuation. In embodiments, drug capsule cap **30** facilitates shaking and/or agitating the drug capsule **20.**

In embodiments, removal of drug capsule cap **30** from drug capsule container **39** actuates other mechanisms and/or has other benefits. In embodiments, removal of drug capsule cap **30** automatically causes removal of a barrier preventing light from passing through one or more viewing windows **54,** which can adversely affect a contained drug and/or medicinal fluid within the drug capsule. In embodiments, removal of cap **30** allows the user to view the medication within (e.g., to confirm it is not adulterated, to confirm dissolving of drug into solution), which is a requirement for many autoinjectors. In embodiments, removal of an adhesive sticker (not shown) serves to prevent light entry during storage and its removal allows viewing through the window once removed. In embodiments, such a sticker may be adhered to drug capsule cap **30,** drug capsule body **50,** and/or cap connector **38,** such that it must be removed before or during the removal of drug capsule cap from the drug capsule body (e.g., to show if it has been tampered with). In embodiments, outer packaging of drug capsule **20** serves to prevent light entry during storage and its removal allows viewing through the window once removed.

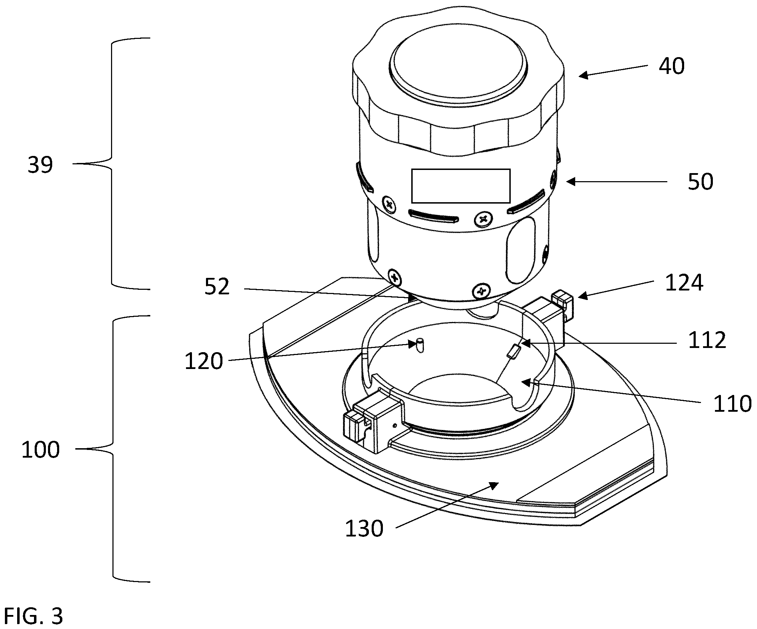

Moving now to FIG. **3****,** an embodiment of part of the present disclosure is shown. Under this embodiment, an improved autoinjector assembly is shown, including drug capsule container **39** and drug delivery holder **100.** In embodiments, drug capsule container **39** is designed such that, once drug capsule cap **30** is removed from drug capsule container **39,** at least part of drug capsule container **39** can then be connected (e.g., placed into) drug delivery holder **100.** In embodiments, drug capsule container **39** may only be connected with (e.g., placed into) drug delivery holder **100** once drug capsule cap **30** is removed from drug capsule container **39.** In embodiments, drug delivery holder **100** further contains one or more holder connection features 124 that interact with drug capsule container **39** to reversibly and/or irreversibly hold the two components together.

In embodiments, when drug capsule container **39** is inserted into drug delivery holder **100,** connection feature **52** on drug capsule body **50** connects and/or otherwise interacts with holder safety feature **120** on drug delivery holder **100.** Under this embodiment, drug capsule container **39** cannot be actuated (i.e., to deploy patient needle **76,** not show in this figure) unless body connection feature **52** and holder safety feature **120** are engaged with each other. Thus, this actuator safety mechanism has the benefit of preventing premature actuation of the improved autoinjector assembly when not appropriately located on the patient (e.g., during device setup, during charging). This further prevents and/or mitigates the chances of a user inadvertently injecting themselves during drug capsule container **39** handling (e.g., prior to insertion into drug delivery holder **100**). In embodiments, holder safety feature **120** and holder connection feature **124** are functionally combined.

In embodiments, drug delivery holder **100** further contains one or more capsule receiving area(s) **110**, which receive drug capsule container **39** (e.g., after drug capsule cap **30** has been removed). In this figure, only one capsule receiving area **110** is shown. However, various embodiments have various numbers of capsule receiving areas (e.g., from one to thirty or more). In embodiments, capsule receiving area **110** additionally has one or more alignment feature(s) **112,** which interact with drug capsule container **39** to ensure appropriate orientation of it when placed into capsule receiving area **110** (e.g., only allowing a specific orientation of placement). In embodiments, drug delivery holder **100** additionally contains patient securing feature **130.** In embodiments, patient securing feature **130** contains an adhesive biocompatible backing that can adhere drug delivery holder **100** to the skin of a patient. In embodiments, patient securing feature **130** includes a strap to facilitate connection to the patient (e.g., for placement around a limb). In embodiments, drug capsule cap **30** is removed completely or partially by placement into capsule receiving area **110** and not manually removed before placement.

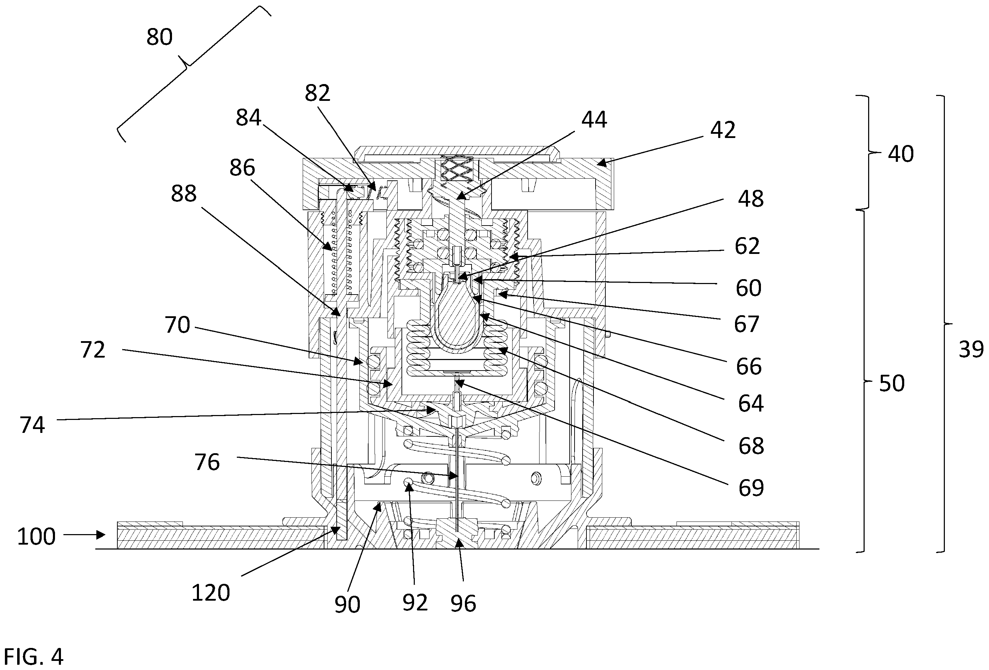

Moving now to FIG. **4**, an embodiment of part of the present disclosure is shown. Under this embodiment, once drug capsule container **39** is appropriately placed in drug delivery holder **100,** and only once appropriately situated there, drug capsule actuator **40** allows the actuation of the device's autoinjector medication delivery mechanism when actuated by the user (e.g., rotated, pressed, flipped, squeezed, twisted, and/or electronically triggered). Actuator release mechanism **80** ensures that drug capsule container **39** can only be actuated when drug capsule container **39** is appropriately place in drug delivery holder **100.** When not fully placed in drug delivery holder **100** (e.g., as shown in FIG **3**), release key **88** is in its distal position, biased to that position by release spring **86.** As release lock **84** is also biased laterally by release lock spring **82,** an interaction between activation grip **42** and release lock **84** ensures that activation grip **42** cannot be actuated (e.g., rotated) and thus the drug capsule cannot be activated in this configuration.

When drug capsule container **39** is appropriately place in drug delivery holder **100** (as shown in FIG **4**), holder safety feature **120** causes release key **88** to move proximally, overcoming release spring **86.** This in turn causes release lock **84** to overcome release lock spring **82** and move medially, thus removing an interaction between activation grip **42** and release lock **84**, which allows activation grip **42** to be actuated (e.g., rotated) in this configuration. Thus, when the drug capsule container **39** is appropriately placed in drug delivery holder **100,** the drug capsule can be activated, although in this embodiment it is not automatically activated until activation grip **42** is separately actuated.

Under some embodiments, drug capsule actuator **40** is located partially or fully on drug delivery holder **100,** instead of as shown fully on drug capsule container **39.**

The actuation apparatus consists of drug capsule actuator **40,** activation grip **42,** activation pin **44,** and pierce pin **48.** To activate drug delivery, in this embodiment the user holds activation grip **42** and rotates it in relation to drug capsule body **50.** This causes activation pin **44** to pierce pressure cylinder **66,** held in place by pressure cylinder retainer **64** and capsule top **60,** which in turn releases pressurized gas into bellows **68,** which is held in place by bellows retainer **67** and activation housing bottom **62.** As the gas expands, it first causes primary container **70** to overcome counterforce from retract spring **92** to move distally, causing patient needle **76** to fully pierce needle septum **96** and subsequently the patient. Under embodiments, needle septum **96** is a resealable sterile barrier.

Once distal motion is halted by interaction of primary container **70** and capsule bottom **90,** the pressure released into bellows **68** causes piston **72** to move distally (as shown in FIG **4**), thus increasing the pressure in injection chamber **74** and thus causing a medicinal fluid to be injected into the patient via patient needle **76,** which is in communication with injection chamber **74.** Once sufficient medicinal fluid has been injected into the patient, piercing needle **69** is then in position to pierce bellows **68.** This causes the pressurized gas within the bellows to escape, which then causes primary container **70** to move proximally due to the now unopposed force of retract spring **92.** This causes patient needle **76** to again retract back past needle septum **96** and into capsule bottom **90.**

Under embodiments, not shown is a second mechanism that ensures auto-retraction even if the needle path is obstructed and the medicinal fluid is not fully injected into the patient. This occurs via a steady leak of gas from bellows **68,** which is a time-based mechanism for auto-retraction.

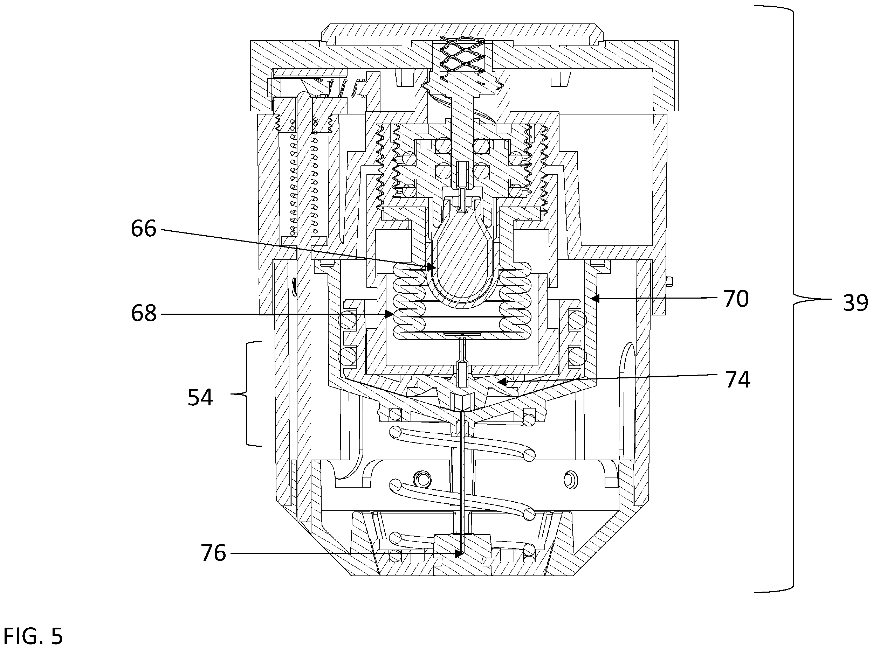

Moving now to FIG. **5**, an embodiment of part of the present disclosure is shown. Under this embodiment, once drug capsule container **39** has previously been actuated (e.g., see FIG **4**), patient needle **76** fully and permanently retracts back into container **39.** This provides the benefit of container **39** functioning as a sharps container for its own needle, as well as ensuring that the needle cannot be used a second time (e.g., on a different patient). In embodiments, this occurs because pressure cylinder **66** has been fully discharged, bellows **68** has been punctured, injection chamber **74** is locked in a collapsed position, and/or another mechanism causes this same outcome. In embodiments, capsule container **39** additionally has a visual indicator viewable by the user that indicates whether it has already been actuated. In embodiments, the visual indicator is viewing window **54** through which the user can visualize the contents of primary container **70.** In embodiments, the visual indicator is one or more separate entities that demonstrate past actuation (e.g., a flag in a window, a permanent change in structure). One benefit of this system is that, once used and retracted, this system ensures that a drug capsule cannot be reused (e.g., on a different patient) or cause a sharps injury (i.e., the needle is fully contained within the device).

Various embodiments of systems, devices, and methods have been described herein. These embodiments are given only by way of example and are not intended to limit the scope of the disclosure. It should be appreciated, moreover, that the various features of the embodiments that have been described may be combined in various ways to produce numerous additional embodiments. Moreover, while various materials, dimensions, shapes, configurations and locations, etc. have been described for use with disclosed embodiments, others besides those disclosed may be utilized without exceeding the scope of the disclosure.

Persons of ordinary skill in the relevant arts will recognize that the subject matter hereof may comprise fewer features than illustrated in any individual embodiment described above. The embodiments described herein are not meant to be an exhaustive presentation of the ways in which the various features of the subject matter hereof may be combined. Accordingly, the embodiments are not mutually exclusive combinations of features; rather, the various embodiments can comprise a combination of different individual features selected from different individual embodiments, as understood by persons of ordinary skill in the art. Moreover, elements described with respect to one embodiment can be implemented in other embodiments even when not described in such embodiments unless otherwise noted.

Although a dependent claim may refer in the claims to a specific combination with one or more other claims, other embodiments can also include a combination of the dependent claim with the subject matter of each other dependent claim or a combination of one or more features with other dependent or independent claims. Such combinations are proposed herein unless it is stated that a specific combination is not intended.

Any incorporation by reference of documents above is limited such that no subject matter is incorporated that is contrary to the explicit disclosure herein. Any incorporation by reference of documents above is further limited such that no claims included in the documents are incorporated by reference herein. Any incorporation by reference of documents above is yet further limited such that any definitions provided in the documents are not incorporated by reference herein unless expressly included herein.

For purposes of interpreting the claims, it is expressly intended that the provisions of 35 U.S.C. § 112(f) are not to be invoked unless the specific terms "means for" or "step for" are recited in a claim.

## Claims

1. An improved autoinjector assembly, comprising:
a drug capsule containing at least one therapeutic medication stored in a dry form, the drug capsule including a drug capsule cap containing a fluid diluent stored separately from the dry form therapeutic medication; and
a drug delivery holder configured to be placed on a body of a patient, the drug delivery holder including one or more capsule receiving areas configured to receive the one or more drug capsules,
wherein the drug capsule is configured to selectively cause the fluid diluent to mix with the dry form therapeutic medication to form a mixture configured to be injected into the body of the patient.

2. The improved autoinjector assembly of claim 1, wherein the drug capsule includes an injection chamber in which the fluid diluent is mixed with the dry form therapeutic medication.

3. The improved autoinjector assembly of claim 2, wherein the drug capsule includes one or more viewing windows that enable the user to view at least part of the injection chamber from outside of the drug capsule.

4. The improved autoinjector assembly of claim 2 or claim 3, wherein the dry form therapeutic medication is initially stored in the injection chamber.

5. The drug capsule of claim 1, wherein the drug capsule further includes a drug capsule body containing the dry form therapeutic medication, a piston, an injection chamber in which the fluid diluent is mixed with the dry form therapeutic medication, and a patient needle in fluid communication with the injection chamber.

6. The improved autoinjector assembly of any preceding claim, wherein the drug capsule delivers a single dose of the mixture as a bolus to an intradermal, subcutaneous, intramuscular, and/or intravenous portion of the body of the patient.

7. The improved autoinjector assembly of claim any preceding claim, wherein the drug capsule further includes an autoinjector medication delivery mechanism configured to inject the mixture into the body of the user and a drug capsule actuator configured to be actuated a user to cause the autoinjector medication delivery mechanism to inject the mixture into the body of the patient.

8. The improved autoinjector assembly of claim 7, wherein the drug capsule further includes an actuator release mechanism that ensures that the drug capsule actuator can only be actuated when the drug capsule is received in the one or more capsule receiving areas of the drug delivery holder.

9. The improved autoinjector assembly of any preceding claim, wherein the drug capsule further includes one or more drug information areas that provide capsule-specific drug information.

10. The improved autoinjector assembly of any preceding claim, wherein the drug delivery holder further includes one or more patient securing features that assist with adhering the drug delivery holder to the patient.

11. The improved autoinjector assembly of any preceding claims, further comprising a plurality of drug delivery capsules.

12. The improved autoinjector assembly of claim 11, wherein the plurality of drug delivery capsules contain different therapeutic medications.

13. A drug delivery capsule for use with the improved autoinjector assembly of any of claims 1-12.

14. A drug delivery holder for use with the improved autoinjector assembly of any of claim 1-12.
